# EUROPÄISCHE PATENTSCHRIFT

(11) **EP 2 953 519 B1**
(45) Veröffentlichungstag und Bekanntmachung des Hinweises auf die Patenterteilung: **14.03.2018**
(21) Anmeldenummer: 14700338.8
(22) Anmeldetag: 07.01.2014
(51) Int. Cl.: F21V 23/06, A61B 1/00, A61B 1/06, A61B 1/12, G02B 23/24, H01R 24/38

(54) **LED-BELEUCHTUNGSMODUL**
LED LIGHTING MODULE
MODULE D'ÉCLAIRAGE À LED

(30) Priorität: 05.02.2013 DE 102013201808
(43) Veröffentlichungstag der Anmeldung: 16.12.2015
(73) Patentinhaber: Richard Wolf GmbH, 75438 Knittlingen (DE)
(72) Erfinder: WEBER, Bernd Claus, 76228 Karlsruhe (DE); HEIMBERGER, Rudolf, 75038 Oberderdingen (DE); SCHRUMPF, Klaus, 76703 Kraichtal-Münzesheim (DE); DETERT, Martina, 81245 München (DE); APP, Thomas, 75059 Zaisenhausen (DE)
(74) Vertreter: Patentanwälte Vollmann & Hemmer
(86) Internationale Anmeldenummer: PCT/EP2014/050130
(87) Internationale Veröffentlichungsnummer: WO 2014/121960

(56) Entgegenhaltungen:
- EP-A1- 2 287 524
- EP-A1- 2 311 366
- US-A1- 2004 227 146
- US-A1- 2005 280 019
- US-A1- 2010 314 986
- US-B1- 6 492 725

## Beschreibung

Die Erfindung betrifft ein medizinisches Instrument mit einem LED-Beleuchtungsmodul mit den im Oberbegriff des Anspruchs 1 angegebenen Merkmalen.
In medizinischen Instrumenten bzw. Geräten, insbesondere in Endoskopen sind Leuchtdioden (LED) zu Beleuchtungszwecken bekannt. Dabei können derartige LED am distalen Ende des Endoskopes platziert werden, sodass auf Lichtleiter im Instrument verzichtet werden kann und das Beleuchtungsmodul direkt dort platziert werden kann, wo die Beleuchtung erforderlich ist. Dies bietet sich sowohl bei starren als auch bei flexiblen Endoskopen an. Problematisch bei der Verwendung von LED ist die anfallende erhebliche Verlustwärme, welche von der LED abgeführt werden muss, um deren Funktion nicht zu beeinträchtigen. Darüber hinaus kann eine Gefährdung des angrenzenden Gewebes durch zu große Erwärmung gegeben sein, sodass die Wärmeabfuhr vorzugsweise so erfolgen muss, dass eine unerwünschte Erwärmung des angrenzenden Gewebes vermieden wird. Ferner ist gleichzeitig eine sicherere Kontaktierung und Fixierung einer LED in dem Endoskop erforderlich.

Im Hinblick auf diese Problematik ist es Aufgabe der Erfindung, ein medizinisches Instrument mit zumindest einem LED-Beleuchtungsmodul bereitzustellen, in welchem eine LED auf einfache Weise elektrisch kontaktiert werden kann und gleichzeitig eine gute Wärmeabfuhr sichergestellt werden kann. Diese Aufgabe wird durch ein medizinisches Instrument mit einem LED-Beleuchtungsmodul mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Gegenstand der Erfindung ist ein medizinisches Instrument mit zumindest einem LED-Beleuchtungsmodul. Das medizinische Instrument ist bevorzugt ein Endoskop. Dabei kann es sich um ein flexibles oder starres Endoskop handeln.
Das LED-Beleuchtungsmodul weist zumindest eine LED auf, welche an einer Proximalseite einen ersten elektrischen Anschlusskontakt und an einer axial entgegengesetzt gerichteten Distalseite einen zweiten elektrischen Anschlusskontakt aufweist. Die LED bzw. der LED-Chip weist somit einen vertikalen Aufbau auf mit nur einer elektrischen Kontaktfläche auf der Chip-Unterseite, nämlich dem ersten proximalwärts gerichteten Anschlusskontakt. Der zweite axial entgegengesetzt gerichtete Anschlusskontakt an der Distalseite ist somit an der Oberseite der LED bzw. des LED-Chips gelegen, welche die die Strahlung emittierende Seite der LED ist. D. h. die axial gerichtete Distalseite der LED, an welcher der zweite elektrische Anschlusskontakt gelegen ist, weist einen strahlungsemittierenden Abstrahlbereich auf. Unter Strahlung ist dabei sowohl das Licht des für das menschliche Auge sichtbaren Spektralbereiches, als auch die Strahlung der für das menschliche Auge nicht sichtbaren angrenzenden kurz- und langwelligen Spektralbereiche zu verstehen. Solche Strahlung kann in medizinischen Instrumenten beispielsweise zu therapeutischen Zwecken, beispielsweise für die fotodynamische Therapie oder beispielsweise für die Autofluoreszenz-Diagnose Verwendung finden.

Erfindungsgemäß ist zum elektrischen Kontaktieren der LED eine elektrisch leitende Hülse vorgesehen. Die Hülse ist vorzugsweise kreiszylindrisch ausgebildet. Die Hülse ist beabstandet von ihrem proximalen Ende mit dem zweiten Anschlusskontakt der LED elektrisch leitend verbunden. So bildet die elektrisch leitende Hülse eine elektrische Verbindung von der Distalseite der LED und dem dort gelegenen zweiten Anschlusskontakt zur Proximalseite, sodass dort dann über die Hülse der distalseitige Anschluss und der proximalseitige Anschluss der LED über eine Anschlussleitung kontaktiert werden können. Auf diese Weise ist eine einfache Kontaktierung der LED möglich. Insbesondere werden größere Kontaktflächen zur elektrischen Kontaktierung geschaffen. Darüber hinaus ermöglicht dieser Aufbau eine gute Wärmeabfuhr. Die Verwendung einer LED mit vertikalem Aufbau, d. h. mit elektrischen Kontakten an der Ober- und Unterseite hat den Vorteil, dass die Unterseite der LED direkt und großflächig mit einer elektrischen Anschlussleitung ohne Zwischenschaltung einer weiteren Leiterplatte kontaktiert werden kann, sodass in proximaler Richtung eine verbesserte Wärmeabfuhr über die Anschlussleitung möglich wird. Die umgebende Hülse kann ebenfalls der Wärmeabfuhr dienen.

Bevorzugt umgibt die Hülse die LED und/oder einen zu dem ersten elektrischen Anschlusskontakt führenden elektrischen Leiter bzw. insbesondere einen diesen isolierenden Mantel umfänglich. Durch diese Ausgestaltung wird sichergestellt, dass der erste elektrische Leiter eine große Kontaktfläche für den ersten elektrischen Anschlusskontakt an der Proximalseite der LED bieten kann und die Hülse relativ dünn und platzsparend im Umfangsbereich angeordnet werden kann, um über die Hülse den zweiten elektrischen Anschlusskontakt an der Distalseite der LED zu kontaktieren. Dazu kann sich die Hülse vollumfänglich oder auch nur in einem umfänglichen Teilbereich an der LED vorbei bis zu deren Distalseite erstrecken, um den zweiten elektrischen Anschlusskontakt zu kontaktieren. So ist es auch möglich, dass sich ausgehend von dem Distalende der Hülse ein Steg bzw. eine Lasche in axialer Richtung an der LED vorbei zu deren zweiten elektrischen Anschlusskontakt erstreckt.

Die Hülse kann die LED und/oder den ersten elektrischen Leiter bzw. insbesondere einen diesen isolierenden Mantel vollumfänglich oder teilumfänglich umgeben. Wenn die Hülse die LED und/oder den ersten elektrischen Leiter vollumfänglich umgibt, bildet sie einen geschlossenen Ring. Wenn sie die LED und/oder den ersten elektrischen Leiter teilumfänglich umgibt, erstreckt sich die Hülse vorzugsweise um einen Umfang größer als 180°, weiter bevorzugt um einen Umfangsbereich größer als 270°, um die LED und/oder den ersten elektrischen Leiter herum. So kann die Hülse in axialer Richtung einen Schlitz aufweisen. Auf diese Weise kann die Hülse mit einer Federwirkung versehen werden, welche es ermöglicht, die Hülse beispielsweise auf einem die LED bzw. den ersten elektrischen Leiter umgebenden isolierenden Mantel klemmend zu halten. Dazu kann die Hülse so ausgebildet sein, dass sie in ihrem entspannten Zustand einen Innendurchmesser aufweist, welcher kleiner ist als der Außendurchmesser, der im Inneren der Hülse aufzunehmenden Bauteile, beispielsweise der Außendurchmesser des isolierenden Mantels. Beim Aufsetzen der Hülse wird diese elastisch verformt und aufgeweitet, so dass sie dann durch die elastischen Rückstellkräfte klemmend am Außumfang der innenliegenden Bauteile gehalten wird.

Weiter bevorzugt ist die Hülse im Bereich ihres distalen Endes mit dem zweiten Anschlusskontakt der LED elektrisch leitend verbunden. Diese elektrisch leitende Verbindung kann direkt am distalen Ende der Hülse gelegen sein, jedoch auch um ein gewisses Maß vom distalen Ende beabstandet sein. Im Bereich des distalen Endes bedeutet dann, dass die elektrische Kontaktierung in axialer Richtung gesehen näher zum distalen Ende als zum proximalen Ende gelegen ist.
Die Hülse weist vorzugsweise eine radial nach innen gerichtete Lasche oder einen radial nach innen gerichteten Vorsprung auf, welche mit dem zweiten Anschlusskontakt der LED elektrisch leitend verbunden sind. Die Lasche ist bevorzugt einstückig mit der Hülse ausgebildet. Die radial nach innen gerichtete Lasche bzw. der radial nach innen gerichtete Vorsprung kann direkt am distalen Ende der Hülse ausgebildet sein, doch auch vom distalen Ende der Hülse beabstandet angeordnet sein. Die radial nach innen gerichtete Lasche bzw. der radial nach innen gerichtete Vorsprung kann in proximaler Richtung vom distalen Ende der Hülse beabstandet sein. Dabei ist die Lasche bzw. der Vorsprung vorzugsweise näher zum distalen Ende der Hülse hin gelegen als zum proximalen Ende der Hülse. Alternativ kann die radial nach innen gerichtete Lasche bzw. der radial nach innen gerichtete Vorsprung auch in distaler Richtung von dem distalen Ende der Hülse beabstandet sein. So kann sich ein Steg bzw. eine Lasche zunächst in distaler Richtung von dem distalen Ende der Hülse wegerstrecken, wobei sich der Vorsprung bzw. die radial nach innen gerichtete Lasche dann von diesem axial gerichteten Steg radial nach innen erstreckt. Dazu kann die Lasche abgewinkelt ausgebildet sein. Die Hülse kann so von der distalen Seite über die LED gestülpt werden bis die Lasche an dem Kontakt an der distalen Stirnseite der LED zur Anlage kommt. Dort kann sie in bekannter geeigneter Weise kontaktiert werden, beispielsweise durch Verlöten oder eine andere geeignete elektrisch leitende Verbindungstechnik.

Bevorzugt ist der erste elektrische Anschlusskontakt der LED, d. h. der an der Proximalseite gelegene elektrische Anschlusskontakt, direkt mit einem ersten elektrischen Leiter zum elektrischen Anschluss der LED verbunden. Dies hat, wie oben ausgeführt, den Vorteil, dass der Wärmeübergang von der LED in diesen elektrischen Leiter verbessert wird und dass keine wärmeisolierenden Bauteile, wie eine Leiterplatte zwischen dem Anschlusskontakt und dem Leiter angeordnet werden müssen. Bevorzugt wird der Leiter direkt großflächig mit der Unterseite der LED kontaktiert. Besonders bevorzugt wird eine Anlage der gesamten proximalen Oberfläche der LED an dem elektrischen Leiter erreicht.

Die Hülse kann an ihrem proximalen Ende oder in ihrem Umfangsbereich direkt oder indirekt mit einem zweiten elektrischen Leiter zum elektrischen Anschluss der LED verbunden werden. Es kann ein solcher Leiter beispielsweise direkt mit der Hülse verlötet sein oder in anderer geeigneter Weise mit dieser in elektrischem Kontakt sein.

Erfindungsgemäß weist die Hülse in axialer Richtung eine größere Länge als die LED auf. So wird sichergestellt, dass die Hülse als elektrischer Leiter bis mindestens zur Proximalseite der LED wirkt und die LED umfangsseitig bevorzugt vollständig umschließt.

Die Hülse kann jedoch auch in Längsrichtung geschlitzt ausgebildet sein, wie oben beschrieben.

So kann die Hülse auch der Abfuhr der am Umfang aus der LED austretenden Wärme dienen. Darüber hinaus kann die Hülse der Führung und dem Schutz der LED dienen. So kann die Hülse die empfindlichen innenliegenden Teile, wie z. B. die LED und deren Kontaktierung während der Lagerung und Montage schützen. Darüber hinaus kann die Hülse in lateraler Richtung als Führungshilfe und in longitaler Richtung als Anschlag bei der Montage dienen. Dazu weist die Hülse bevorzugt im Verhältnis zu ihrem Durchmesser eine ausreichende Länge auf. Darüber hinaus kann die Hülse als Schnittstelle für die Fixierung des LED-Beleuchtungsmoduls im Inneren eines medizinischen Gerätes, insbesondere eines Endoskops fungieren. In distaler Richtung steht die Hülse vorzugsweise nicht oder im Wesentlichen nicht über die Distalseite der LED vor, sodass die Hülse mit der LED im Inneren eines Endoskops möglichst nahe an einem Austrittsfender platziert werden kann. Vorzugsweise steht die Hülse am distalen Ende lediglich um die Dicke der Lasche, welche zur Kontaktierung des zweiten elektrischen Anschlusskontaktes dient, über das distale Ende der LED vor.

Der erste elektrische Anschlusskontakt, d. h. der an der proximalen Seite der LED gelegene Anschlusskontakt ist mit einer Gegenkontaktfläche des ersten elektrischen Leiters zum elektrischen Anschluss der LED elektrisch leitend verbunden, wobei die Gegenkontaktfläche eine gleiche oder größere Fläche als der erste Anschlusskontakt aufweist. Auf diese Weise kann ein direkter Wärmeübergang über die gesamte Anschlusskontaktfläche in den angeschlossenen elektrischen Leiter erreicht werden, wodurch die Wärmeabfuhr in proximaler Richtung über den elektrischen Leiter verbessert wird. Der Anschlusskontakt kann mit der Gegenkontaktfläche beispielsweise verlötet oder elektrisch leitend verklebt sein.

So kann beispielsweise der erste Anschlusskontakt eine rechteckige, insbesondere quadratische Kontaktfläche aufweisen und die Gegenkontaktfläche eine runde, insbesondere kreisförmige Form aufweisen, wobei der Durchmesser der Gegenkontaktfläche weiter bevorzugt zumindest der Länge einer Diagonalen der rechteckigen bzw. quadratische Kontaktfläche entspricht. So wird sichergestellt, dass der Anschlusskontakt vollständig an der Gegenkontaktfläche anliegen kann.

Gemäß einer weiteren bevorzugten Ausführungsform wird ein Koaxialkabel zum elektrischen Anschluss der LED verwendet, von welchem ein Innenleiter einen ersten elektrischen Leiter bildet, welcher mit dem ersten Anschlusskontakt der LED verbunden ist und ein Außenleiter elektrisch leitend mit der Hülse verbunden ist. Mit einem solchen Koaxialkabel kann eine sehr große Querschnittsfläche der Leiter zur Verfügung gestellt werden, welche eine gute Wärmeabfuhr in proximaler Richtung ermöglicht. Insbesondere kann der Innenleiter des Koaxialkabels mit einem derart großen Querschnitt ausgebildet werden, dass er der Wärmeabfuhr von der LED in proximaler Richtung dienen kann. Bevorzugt hat der Innenleiter eine Querschnittsfläche, welche mindestens der Querschnittsfläche des ersten elektrischen Anschlusskontaktes entspricht, sodass der erste elektrische Anschlusskontakt vollflächig auf der Stirnseite des Innenleiters des Koaxialkabels anliegen kann. Die erfindungsgemäß vorgesehene Hülse vereinfacht die Kontaktierung eines solchen Koaxialkabels, da der Innenleiter des Koaxialkabels so direkt mit dem proximalseitigen Anschlusskontakt der LED verbunden werden kann, während die Hülse eine Kontaktierung des Außenleiters des Koaxialkabels mit dem Anschlusskontakt an der distalseitigen Oberfläche der LED in einfacher Weise ermöglicht.

Gemäß einer weiteren Ausführungsform kann der die LED umgebende Innenraum der Hülse mit einem vorzugsweise für eine von der LED emittierte Strahlung transparenten Füllmaterial gefüllt sein. Die Hülse bildet dabei einen Behälter oder ein Gefäß, welches das Füllmaterial aufnimmt. Dies ist besonders dann von Vorteil, wenn das Füllmaterial in flüssiger Form eingefüllt wird. Dieses Füllmaterial kann zum einen der Fixierung der LED im Inneren der Hülse dienen, und zum anderen die Wärmeabfuhr von der LED zu der Hülse verbessern. Das Füllmaterial kann ferner auch zur Stabilität und Festigkeit der Hülse und des LED-Beleuchtungsmoduls insgesamt beitragen. So weist die mit dem Füllmaterial gefüllte Hülse gegenüber äußeren mechanischen Einwirkungen eine größere Festigkeit auf, wenn ein Füllmaterial verwendet wird, welches nach dem Aushärten eine entsprechend hohe Festigkeit aufweist. Darüber hinaus kann es an der Austrittsseite der LED, an welcher die Strahlung austritt wenn es für die von der LED emittierte Strahlung transparent ausgebildet ist, eine optische Beeinflussung der Strahlung ermöglichen, beispielsweise durch eine geeignete Wahl des Brechungsindex eine gewünschte Brechung der Strahlung beim Übergang bei der LED in das Füllmaterial und gegebenenfalls von dem Füllmaterial zu einem Austrittsfenster oder weiteren optischen Bauteilen ermöglichen. Ferner kann das Füllmaterial, beispielsweise eine Vergussmasse, welche distalseitig der LED gelegen ist, ein Konvertermaterial enthalten, welches beispielsweise einen Wellenlängenbereich des von der LED emittierten Lichtes verschiebt, erweitert oder ändert. Des Weiteren können bei geeigneter Wahl des Füllmaterials die LED und die elektrischen Anschlusskontakte oder zumindest ein elektrischer Anschlusskontakt durch das Füllmaterial vor Feuchtigkeit geschützt werden. Dabei ist das Füllmaterial bevorzugt so zu wählen, dass es den entsprechenden Aufbereitungsverfahren für medizinische Geräte, z. B. Autoklavieren, standhält.

Gemäß einer weiteren bevorzugten Ausführungsform ist die Hülse an ihrem Außenumfang von einem thermisch isolierenden Mantel umgeben. Dadurch wird eine Wärmeableitung bzw. Wärmeabstrahlung von der Hülse nach außen vermieden, sodass eine Erwärmung der die LED und die Hülse umgebenden Bereiche verringert wird. Die Wärmeableitung wird vorzugsweise so optimiert, dass der größte Teil der Wärme in proximaler Richtung von der LED auf die elektrische Anschlussleitung und/oder von der Hülse auf die proximalseitig anschließenden Bauteile übertragen wird.

Der thermisch isolierende Mantel kann beispielsweise aus einem Kunststoffmaterial gebildet sein, welches direkt auf die Hülse aufgebracht ist oder die Hülse umgibt.

Die Hülse kann gemäß einer weiteren Ausführungsform der Erfindung so angeordnet und ausgestaltet sein, dass ein distales Ende der Hülse von der Distalseite der LED in proximaler Richtung beabstandet ist. D. h. in distaler Richtung erstreckt sich die Hülse dann nicht bis zum distalen Ende der LED. In diesem Ausführungsbeispiel kann die Kontaktierung des zweiten elektrischen Anschlusskontaktes der LED dann über einen Steg bzw. eine Lasche erfolgen, welche sich in axialer Richtung von dem distalen Ende der Hülse an der LED vorbei bis zu deren Distalseite erstreckt und so den zweiten elektrischen Anschlusskontakt an der Distalseite der LED kontaktieren. Dazu kann sich ausgehend von dieser Lasche bzw. diesem Steg eine Lasche bzw. ein Vorsprung radial nach innen zu dem zweiten elektrischen Anschlusskontakt erstrecken. Der sich in distaler Richtung von der Hülse wegerstreckende Steg kann dazu beispielsweise an seinem Distalende abgewinkelt sei.

Gemäß einer alternativen Ausführungsform der Erfindung kann sich die Hülse axial über das Distalende der LED hinaus erstrecken und am distalen Ende der Hülse kann im Inneren der Hülse ein Fenster angeordnet sein. Die Hülse bildet somit einen Träger für das Fenster, welches dann distalseitig der LED gelegen ist. Ein ggf. zwischen der LED und dem Fenster vorhandener Freiraum kann, wie oben beschrieben, durch ein geeignetes transparentes Füllmaterial ausgefüllt sein. Dieses Füllmaterial kann, wie oben beschrieben, gezielte optische Eigenschaften aufweisen oder beispielsweise auch ein Konvertermaterial enthalten. Das Fenster, welches insbesondere aus einen amorphen Werkstoff, z. B. aus Glas oder einem kristallinen Werkstoff z. B. Saphir gefertigt ist, weist einen Außenumfang auf, welcher zum Innenumfang bzw. Innendurchmesser der Hülse korrespondiert und somit bevorzugt dicht am Innenumfang der Hülse anliegen kann. Das Fenster kann mit der Hülse verklebt oder verlötet oder in anderer Weise vorzugsweise dicht verbunden sein. So bildet das Fenster eine axialseitige Abdichtung des LED-Beleuchtungsmoduls. So kann im LED-Beleuchtungsmodul selber eine dichte Kapselung der elektrischen Bauteile wie der LED und der elektrischen Anschlusskontakte erreicht werden. Dies bietet zum einen einen Schutz dieser Bauteile vor der endgültigen Montage in einem medizinischen Instrument bzw. Gerät, schützt zum anderen die Bauteile aber auch im medizinischen Instrument später vor eindringender Feuchtigkeit. Darüber hinaus trägt das Fenster zur Stabilität des LED-Beleuchtungsmoduls und der Hülse insbesondere gegen laterale Krafteinwirkung bei. So ist das LED-Modul vor und während der Montage besser gegen Beschädigungen geschützt. Das Fenster liegt in der Hülse bevorzugt distalseitig eines sich von der Hülse radial nach innen erstreckenden Vorsprunges bzw. einer sich radial nach innen erstreckenden Lasche zur Kontaktierung des distalseitgen Anschlusskontaktes der LED.

In einer weiteren bevorzugten Ausführungsform kann die Hülse von einer weiteren Außenhülse umgeben sein, welche sich axial über ein distales Ende der vorangehend beschriebenen Hülse hinaus erstreckt und in derem Innern am distalen Ende ein Fenster angeordnet ist. Diese Außenhülse kann ebenfalls elektrisch leitend oder auch elektrisch isolierend ausgebildet sein. Das Fenster weist einen Außendurchmesser bzw. Außenumfang auf, welcher zum Innendurchmesser bzw. Innenumfang der Außenhülse korrespondiert, so dass das Fenster mit seinem Außenumfang vorzugsweise dicht am Innenumfang der Außenhülse anliegt. Das Fenster, welches vorzugsweise aus Glas oder einem kristallinen Medium ausgebildet ist, kann mit der Außenhülse verklebt oder verlötet oder in anderer Weise vorzugsweise dicht verbunden sein. Die Außenhülse mit dem Fenster kann vom distalen Ende her über die LED umgebende elektrisch leitende Hülse gestülpt werden. Bevorzugt wird die Außenhülse dann dicht mit der innenliegenden Hülse verbunden, beispielsweise verlötet oder verklebt. Diese Ausgestaltung hat den Vorteil, dass vor dem Aufsetzen der Außenhülse die Hülse zur Kontaktierung der LED mit dem distalseitigen Anschlusskontakt der LED verlötet werden kann und die Hülse ggf. an der Distalseite der LED mit einem Füllmaterial gefüllt werden kann. Dies kann dann von der offenen Distalseite der Hülse her geschehen. Anschließend wir die Hülse durch Überstülpen der Außenhülse mit dem Fenster dicht verschlossen. Dabei weist die Außenhülse vorzugsweise eine solche axiale Länge auf, dass Ausnehmungen oder Öffnungen in der Innenhülse außenumfänglich von der Außenhülse umschlossen werden. Solche Öffnungen oder Ausnehmungen können beispielsweise durch eine radial nach innen gebogene Lasche der inneren Hülse zur Kontaktierung des distalseitigen Anschlusskontaktes der LED gebildet sein. Auch bei dieser Ausführungsform wird bevorzugt ein vollständig dichtes und gekapseltes LED-Beleuchtungsmodul geschaffen, welches vor Feuchtigkeit und Beschädigungen vor und während der Montage geschützt ist. Das Fenster und die Außenhülse tragen zur weiteren Stabilisierung des LED-Beleuchtungsmoduls bei.

Die vorangehend beschriebenen Fenster, welche in der Hülse oder Außenhülse angeordnet werden können, bilden beim Einbau des LED-Beleuchtungsmoduls in ein medizinisches Gerät bzw. ein Endoskop vorzugsweise das distalseitige äußere Fenster des Beleuchungsmoduls. D. h. im Endoskop muss kein weiteres Fenster an der Distalseite vorgesehen werden. Die Hülse oder ggf. die Außenhülse werden dann bevorzugt dicht mit einer umgebenden Wandung des Instrumentes bzw. Endoskops verbunden, beispielsweise verklebt.

Besonders bevorzugt ist das LED-Beleuchtungsmodul der Erfindung, wie es vorangehend beschrieben wurde, zum Einsatz in einem medizinischen Gerät und insbesondere in einem Endoskop ausgebildet. In solchen Geräten eignet sich das erfindungsgemäße LED-Beleuchtungsmodul insbesondere zur Anordnung am distalen Ende des Gerätes. Dies ist bei einem Endoskop dasjenige Ende, welches in den Körper eingeführt wird. In diesem Bereich ist häufig eine Erwärmung zu vermeiden, um eine Beschädigung des umgebenden Gewebes zu verhindern. Insofern ist es bei diesen Geräten wünschenswert, die Wärme gezielt in proximaler Richtung aus dem Untersuchungsgebiet heraus abführen zu können. Die direkte Kontaktierung der LED mit einer elektrischen Anschlussleitung und die beschriebene Verwendung der Hülse zur Kontaktierung ermöglichen dabei eine optimierte Wärmeabfuhr über die Anschlussleitung in proximaler Richtung. Dies ist jedoch nicht nur bei medizinischen Endoskopen von Vorteil, sondern kann auch bei technischen Endoskopen von Nutzen sein, sodass die Verwendung nicht auf medizinische Endoskope beschränkt ist.

Gemäß einer bevorzugten Ausführungsform des medizinischen Geräts liegt die Hülse des LED-Beleuchtungsmoduls mit ihrer distalen Stirnseite an zumindest eine Anschlagfläche im inneren eines Kopfes des Endoskopes an. So wird die Hülse in axialer Richtung mit der im Inneren angeordneten LED im Inneren des Endoskopes fixiert. Die Anschlagfläche kann dabei von einem Sicht- oder Austrittsfenster am distalen Ende des Kopfes des Endoskopes gebildet werden.

Nachfolgend wird die Erfindung beispielhaft anhand der beigefügten Figuren beschrieben. In diesen zeigt:
- Fig. 1: schematisch in einer Schnittansicht den Aufbau eines erfindungsgemäßen LED-Beleuchtungsmoduls,
- Fig. 2: schematisch die Anordnung eines LED-Beleuchtungsmoduls gemäß Fig. 1 in einem flexiblen Endoskop,
- Fig. 3: eine schematische Schnittansicht der Anordnung eines erfindungsgemäßen LED-Beleuchtungsmoduls in einem Endoskop,
- Fig. 4: eine Draufsicht auf das LED-Beleuchtungsmodulgemäß Fig. 3 aus distaler Richtung,
- Fig. 5: eine weitere mögliche Anordnung eines erfindungsgemäßen LED-Beleuchtungsmoduls in einem Endoskop in einer schematischen Schnittansicht,
- Fig. 6: eine Draufsicht auf das Beleuchtungsmodul gemäß Fig. 5,
- Fig. 7: eine weitere mögliche Anordnung eines LED-Beleuchtungsmodules in einem Endoskop in einer schematischen Schnittansicht,
- Fig. 8: eine weitere mögliche Anordnung eines LED-Beleuchtungsmodules in einem Endoskop in einer schematischen Schnittansicht,
- Fig. 9: eine weitere mögliche Anordnung eines LED-Beleuchtungsmodules in einem Endoskop in einer schematischen Schnittansicht,
- Fig. 10 - 12: drei mögliche Ausführungsformen einer Hülse für ein erfindungsgemäßes LED-Beleuchtungsmodul,
- Fig. 13, 14: zwei weitere mögliche Anordnungen eines LED-Beleuchtungsmoduls in einem Endoskop in einer schematischen Schnittansicht,
- Fig. 15: eine Draufsicht auf ein LED-Beleuchtungsmodul gemäß einer weiteren Ausführungsform der Erfindung aus distaler Richtung, und
- Fig. 16: eine Schnittansicht einer weiteren möglichen Anordnung eines LED-Beleuchtungsmoduls in einem Endoskop.

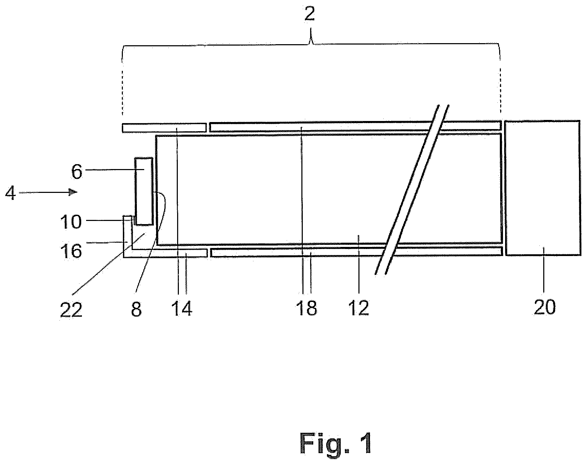

Anhand der schematischen Schnittansicht in Fig. 1 wird zunächst der grundsätzliche Aufbau eines erfindungsgemäßen LED-Beleuchtungsmodules beschrieben. Das LED-Beleuchtungsmodul weist an seinem distalen Ende eine LED bzw. einen LED-Chip 6 auf, welcher Strahlung bzw. Licht in distaler Richtung aussendet. Der LED-Chip 6 weist einen vertikalen Aufbau auf, d. h. er hat einen ersten elektrischen Anschlusskontakt 8 an seiner proximalen Seite und einen zweiten elektrischen Anschlusskontakt 10 an seiner distalen Seite, welche auch die Austrittsseite für die Strahlung bzw. das von dem LED-Chip 6 emittierte Licht bildet. Der erste Anschlusskontakt 8 ist mit einem ersten elektrischen Leiter 12 verbunden. Zu erkennen ist, dass eine großflächige Kontaktierung zwischen dem ersten Anschlusskontakt 8 und dem ersten elektrischen Leiter 12 möglich ist. Der LED-Chip 6 liegt mit seinem ersten Anschlusskontakt 8 vollflächig an der Stirnseite des ersten elektrischen Leiters 12 an, sodass hier ein guter Wärmeübergang in proximaler Richtung erreicht wird. Der zweite elektrische Anschlusskontakt 10 des LED-Chips 6 wird von einer Hülse 14 kontaktiert. Die Hülse 14 ist elektrisch leitend, beispielsweise aus Metall ausgebildet und umgibt den LED-Chip 6 umfänglich. Am distalen Ende der Hülse 14 weist diese eine radial nach innen gerichtete Lasche 16 auf, welche den zweiten elektrischen Anschlusskontakt 10 an der distalen Seite des LED-Chips 6 kontaktiert.

Am proximalen Ende ist die Hülse 14 mit einem zweiten elektrischen Leiter 18 verbunden. Der zweite elektrische Leiter 18 bildet die Rückleitung, während der erste elektrische Leiter 12 die Hinleitung bildet. Beide sind in hier nicht näher gezeigter Weise elektrisch gegeneinander isoliert. Hier ist eine koaxiale Anordnung gezeigt. Bevorzugt sind der erste elektrische Leiter 12 und der zweite elektrische Leiter 18 sowie die zugehörigen elektrischen Isolierungen flexibel ausgebildet, sodass die Beleuchtungseinheit 2 in einem flexiblen Endoskop Verwendung finden kann. Der erste elektrische Leiter 12 und der zweite elektrische Leiter 18 sind an ihrem proximalen Ende mit einer hier schematisch dargestellten Energiequelle 20 verbunden.

Der den LED-Chip 6 umgebende Freiraum 22 kann mit einem geeigneten Füllmaterial gefüllt sein. Sofern sich dieses Füllmaterial auch über die distale Oberfläche des LED-Chips 6 erstreckt, ist dieses bevorzugt für die von der LED 6 emittierte Strahlung bzw. das emittierte Licht transparent ausgebildet. Das Füllmaterial kann aber auch ein Konvertermaterial enthalten, welches zumindest einen Teil der von der LED 6 emittierten Strahlung absorbiert und in Strahlung, welche dem längerwelligen Spektralbereich zuzuordnen ist, umwandelt. Durch die geeignete Wahl des Brechungsindexes können hier optische Beeinflussungen der emittierten Strahlung bzw. des emittierten Lichtes, insbesondere beim Übergang in ein sich distalseitig anschließendes Austrittsfenster erreicht werden. Beispielsweise kann die Strahlung in geeigneter Weise gebrochen werden. Der Freiraum 22 kann beispielsweise mit Silikon, Epoxidharz oder einem ähnlichen Füllmaterial gefüllt sein, welches gewünschte optische Eigenschaften aufweist, darüber hinaus den LED-Chip schützt und in der Hülse fixiert. Alternativ kann der Freiraum 22 auch mit Luft gefüllt sein.

Die Verbindungen des LED-Chips 6 mit dem ersten elektrischen Leiter 12 und der Hülse 14 kann in bekannter Weise, beispielsweise durch Verlöten oder Leitkleber erfolgen. Die Hülse 14 ist vorzugsweise dünnwandig ausgebildet, um die laterale Ausdehnung des Beleuchtungsmoduls 2 gering zu halten. Die Lasche 16 weist eine Größe auf, welche ausreicht, den Anschlusskontakt 10 zu kontaktieren, ist vorzugsweise jedoch so klein und so schlank gebildet, dass sie die Licht- bzw. Strahlungsemission des LED-Chips 6 idealerweise gar nicht oder zumindest so wenig wie möglich einschränkt. Die Hülse 14 überragt den LED-Chip 6 in distaler und insbesondere auch in proximaler Richtung, sodass der LED-Chip 6 geschützt im Inneren der Hülse angeordnet ist. Die Hülse 14 kann so auch der Fixierung und Positionierung des LED-Chips 6 in einem medizinischen Instrument, beispielsweise einem Endoskop dienen. Der Überstand in distaler Richtung ist jedoch so gering gewählt, dass sich gerade die Lasche 16 über die distale Seite des LED-Chips 6 erstrecken kann. Dadurch kann der Abstand des LED-Chips zu einem sich distalseitig anschließenden Fenster oder weiteren optischen Bauteilen möglichst gering gehalten werden.

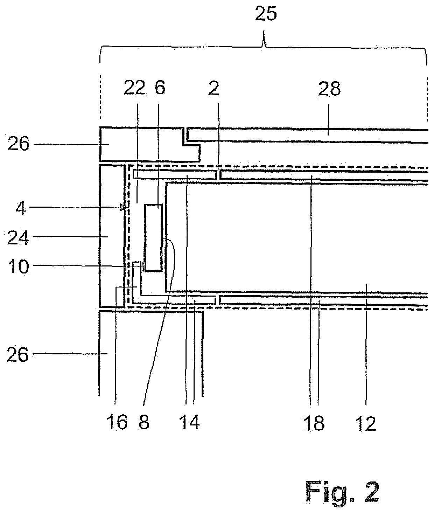

Fig. 2 zeigt den möglichen Einbau des LED-Beleuchtungsmoduls 2 in ein medizinisches Gerät, hier beispielsweise ein flexibles Endoskop. Das in Fig. 1 gezeigte LED-Beleuchtungsmodul 2 liegt in Fig. 2 mit seinem distalen Ende 4 an einem Fenster 24, welches das Austrittsfenster am distalen Ende des Endoskops bildet, an. Das Fenster 24 ist beispielsweise als Glasplättchen oder transparentes Kunststoffplättchen ausgebildet. Das Fenster 24 ist in die stirnseitige Wandung 26 eines Endoskopkopfes bzw. Endoskopköpfchens eingebettet. Das Endoskopköpfchen ist am distalen Ende eines Endoskopschlauches bzw. Endoskopschaftes 28 angeordnet, in dessen Inneren das LED-Beleuchtungsmodul 2 aufgenommen ist. Es ist zu erkennen, dass die Hülse 14 geeignet ist, den zuvor in der Hülse 14 eingebetteten LED-Chip 6 in der stirnseitigen Wandung 26 des Endoskopköpfchens in einer geeigneten Ausnehmung zu fixieren und direkt hinter dem Fenster 24 zu positionieren.

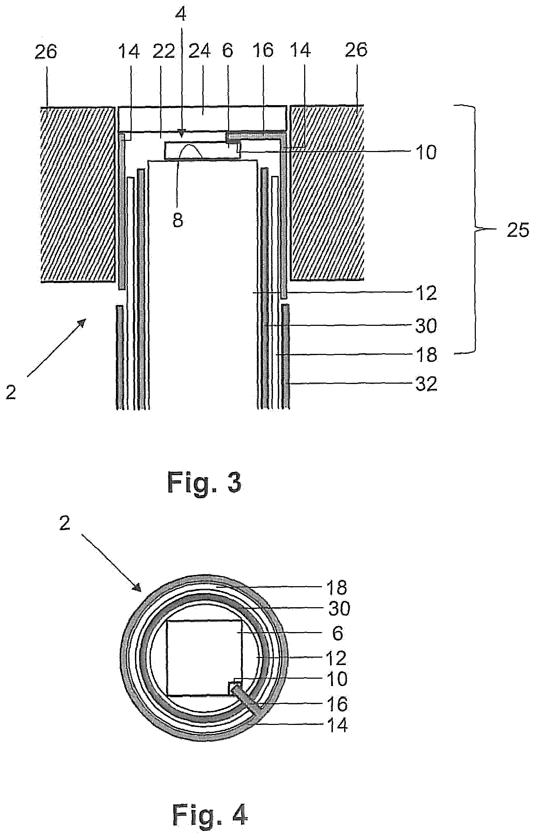

Fig. 3 zeigt eine konkretere Ausgestaltung der Anordnung gemäß Fig. 2, bei welcher zusätzlich die erforderlichen Isolierungen in der elektrischen Anschlussleitung gezeigt sind. Der erste elektrische Leiter 12 bildet die Seele eines Koaxialkabels und ist von einer ersten elektrischen Isolierung 30 umfänglich umgeben. Die erste elektrische Isolierung 30 wiederum ist umfänglich von dem zweiten elektrischen Leiter 18 umgeben, welcher den Schirm des Koaxialkabels bildet. Der zweite elektrische Leiter ist seinerseits umfänglich von einer zweiten elektrischen Isolierung 32 umgeben. Zu erkennen ist, dass der erste elektrische Leiter 12 einen wesentlich größeren Querschnitt aufweist als der zweite elektrische Leiter 18. So kann der erste elektrische Leiter 12 vollflächig oder nahezu vollflächig an der proximalen Seite des LED-Chips 6 anliegen und den Großteil der anfallenden Verlustwärme in proximaler Richtung durch das Koaxialkabel abführen. Der größere Querschnitt bildet einen geringeren thermischen Widerstand. Die erste und die zweite Isolierung 30 und 32 sowie der zweite elektrische Leiter 18 werden jedoch in radialer bzw. lateraler Richtung bevorzugt möglichst dünn ausgebildet, um die laterale Ausdehnung der Beleuchtungseinheit 2 gering zu halten. Darüber hinaus wird ein geringer thermischer Widerstand des Koaxialkabels in lateraler Richtung bewirkt, sodass die Wärme aus dem Kabel in den Umfangsbereich abgegeben werden kann. So kann die von dem LED-Chip 6 proximalwärts abgeführte Wärme dann in radialer bzw. lateraler Richtung aus dem sich proximalseitig anschließenden Anschlusskabel austreten. Das distale Ende des Instrumentes wird jedoch so geringer thermisch belastet bzw. erwärmt. Der zweite elektrische Leiter 18 ist in das Innere der Hülse 14 hineingeführt und dort an der Innenseite mit der Hülse kontaktiert. Die Hülse 14 stellt dann, wie vorangehend beschrieben, den elektrischen Kontakt zu dem Anschlusskontakt 10 an der distalen Seite des LED-Chips 6 her.

Fig. 4 zeigt eine Draufsicht auf das LED-Modul 2 im Inneren des in Fig. 3 gezeigten Endoskopkopfes. Es ist zu erkennen, dass die Hülse 14 den LED-Chip 6 umfänglich umgibt und dass nur die schmale Lasche 16 den LED-Chip 6 an einer Ecke distalseitig überdeckt, um den Anschlusskontakt 10 zu kontaktieren. Ferner ist zu erkennen, dass der quadratische LED-Chip 6 vollständig auf der runden distalen Stirnseite des ersten elektrischen Leiters 12, d. h. der Seele des Koaxialkabels aufliegt. Dazu hat der erste elektrische Leiter 12 einen Durchmesser, welcher im Wesentlichen der Diagonale des LED-Chips 6 entspricht.

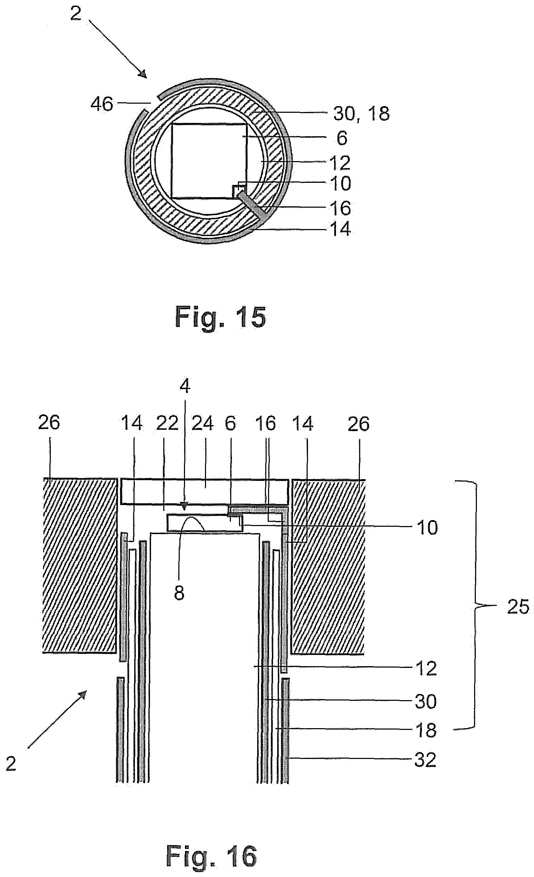

Eine Abwandlung der Ausführungsform gemäß Fig. 3 ist in Fig. 16 gezeigt. Die in Fig. 16 gezeigte Ausführungsform unterscheidet sich von der Ausgestaltung gemäß Fig. 3 darin, dass die Hülse 14 sich nicht umfänglich der LED 6 bis zu dem Fenster 24 vollumfänglich erstreckt. Vielmehr endet in diesem Ausführungsbeispiel die Hülse 14 in Längsrichtung im Wesentlichen mit dem ersten elektrischen Leiter 12. Lediglich die Lasche 16 erstreckt sich zunächst in axialer Verlängerung der Hülse 14 und dann gewinkelt radial nach innen über das distale Stirnende der Hülse 14 hinaus. So erstreckt sich die Lasche 16 stegförmig der LED 6 an einer Umfangsseite der LED vorbei bis zu deren distalen Stirnseite, wo dann der radial nach innen abgewinkelte Teil der Lasche 16 den zweiten Anschlusskontakt 10 kontaktiert. Im Übrigen entspricht die Ausgestaltung gemäß Fig. 16 der Ausgestaltung gemäß Fig. 3, sodass bzgl. weiterer Merkmale auf die vorangehende Beschreibung verwiesen wird.

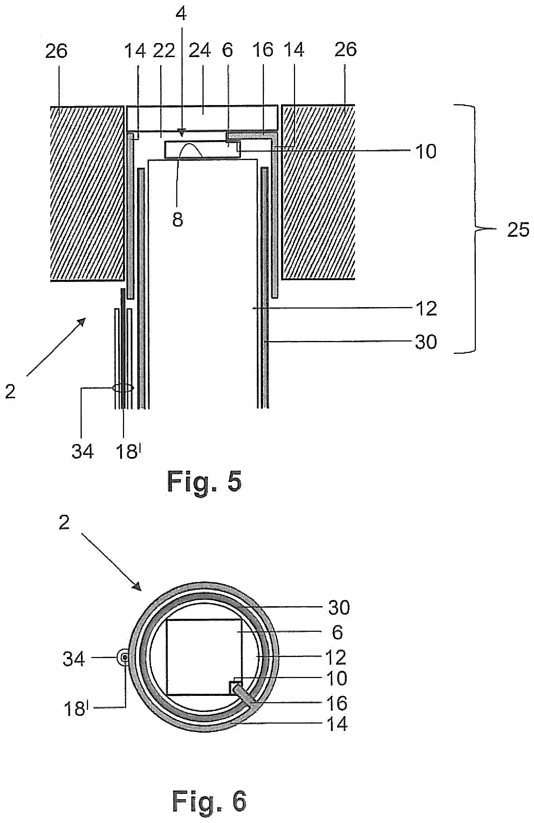

Fig. 5 zeigt einen alternativen Aufbau eines Endoskopköpfchens 25, insbesondere eines flexiblen Endoskops. Auch dort ist das LED-Beleuchtungsmodul 2 in einer Ausnehmung der stirnseitigen Wandung 26 direkt angrenzend an ein Fenster 24 angeordnet. Die Hülse 14 stößt dabei mit ihrem distalen Ende und insbesondere der Lasche 16 direkt an die proximale Seite des Fensters 24 an, wie es auch bei den Ausführungsformen gemäß Fig. 2 und 3 gezeigt ist. Auch bei der Ausführungsform gemäß Fig. 5 ist der LED-Chip 6 an seiner proximalen Seite, d. h. seinem ersten elektrischen Anschlusskontakt 8 mit der Stirnseite eines ersten elektrischen Leiters 12 kontaktiert. Dabei weist der erste elektrische Leiter 12 wiederum einen Durchmesser auf, welcher so groß ist, dass er die komplette oder nahezu komplette proximale Seite des LED-Chips 6 überdeckt und hier für eine gute Wärmeübertragung bei geringem thermischem Widerstand sorgt. Der elektrische Leiter 12 ist von der ersten Isolierung 30 umgeben und kann bei dieser Ausführungsform als einfacher Litzenleiter ausgebildet sein. Der zweite elektrische Leiter 18' ist in einem separaten elektrischen Kabel 34 angeordnet und an der Umfangsseite mit dem proximalen Ende der Hülse 14 verbunden. Die Verbindung kann ebenfalls durch Löten, Klemmen, Crimpen, Leitkleben oder ähnliches erfolgen. Die Anordnung gemäß den Figuren 5 und 6 hat gegenüber der Anordnung gemäß Fig. 3 und 4 den Vorteil, dass durch den Verzicht der zweiten Isolierung 32 und des zweiten elektrischen Leiters 18 im Umfangsbereich des ersten elektrischen Leiters 12, d. h. den Verzicht auf den koaxialen Aufbau, der Durchmesser bzw. die laterale Ausdehnung der elektrischen Anschlussleitung verringert werden kann. So kann der elektrische Leiter 12 mit größerem Querschnitt oder Durchmesser ausgebildet werden, wodurch der thermische Widerstand weiter reduziert werden kann und die Wärmeabfuhr in proximaler Richtung verbessert werden kann. Aufgrund der geringeren lateralen Ausdehnung eignet sich diese Ausführungsform insbesondere bei Endoskopen mit kleinem zur Verfügung stehendem Bauraum.

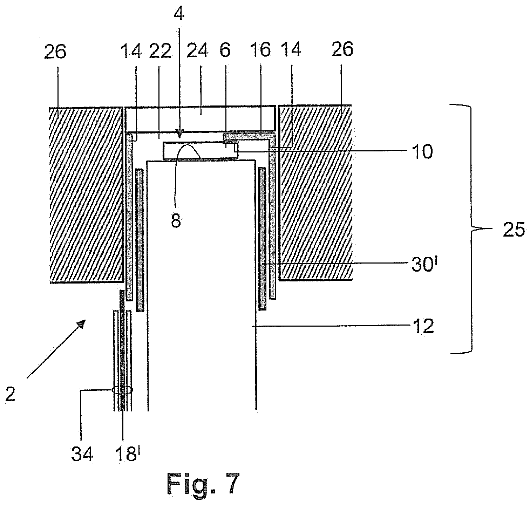

Diese Ausgestaltung wird noch weiter bei der in Fig. 7 dargestellten Ausführungsform optimiert, bei welcher auf eine den Hinleiter bzw. den ersten elektrischen Leiter 12 über die gesamte Länge umgebende Isolierung verzichtet wird. Dort ist eine erste elektrische Isolierung 30' umfänglich des ersten elektrischen Leiters 12 nur innerhalb der Hülse 14 vorgesehen, um einen Kurzschluss zwischen der Hülse 14 und dem ersten elektrischen Leiter 12 zu vermeiden. Jenseits des proximalen Endes der Hülse ist jedoch auf eine umfängliche Isolierung des ersten elektrischen Leiters 12 verzichtet worden. Ein Kurzschluss mit dem Rückleiter 18' wird hier nur durch die Isolierung des den Rückleiter 18' enthaltenden Kabels 34 verhindert. Dies hat den Vorteil, dass Verlustwärme aus dem ersten elektrischen Leiter 12 in dem Bereich, welcher frei von einer umfänglichen Isolierung ist, besser in radialer Richtung abstrahlen bzw. abgeleitet werden kann.

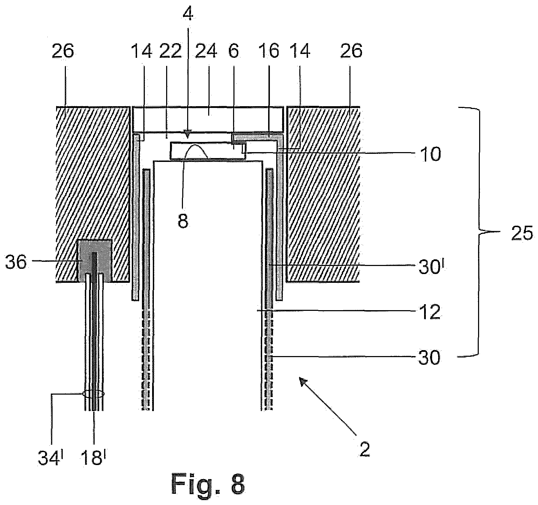

Fig. 8 zeigt eine weitere Ausführungsform, welche eine Variante der in den Fig. 5 - 7 gezeigten Ausführungsformen darstellt. Die Ausgestaltung des ersten elektrischen Leiters 12 entspricht dabei den Ausgestaltungen gemäß Fig. 5 - 7, wobei wahlweise die erste Isolierung 30, 30' über das proximale Ende der Hülse 14 hinaus verlängert sein kann, wie in Fig. 5 gezeigt, oder aber hinter dem proximalen Ende der Hülse 14 enden kann, wie in Fig. 7 gezeigt ist. Dies ist durch den gestrichelten Verlauf der ersten Isolierung 30 in Fig. 8 gezeigt. Im Unterschied zu den Ausführungsformen gemäß Fig. 5 - 7 ist in der Ausführungsform gemäß Fig. 8 das Kabel 34' nicht direkt mit der Hülse 14 verbunden, sondern mit der stirnseitigen Wandung 26 des Endoskopköpfchens 25. Dazu kann der zweite elektrische Leiter 18' im Inneren eines Sackloches 36 in der stirnseitigen Wandung 26 mit dieser verlötet oder in anderer geeigneter Weise elektrisch verbunden sein. Die Wandung 26 ist elektrisch leitend ausgebildet und mit dem Außenumfang der Hülse 14 in elektrischem Kontakt.

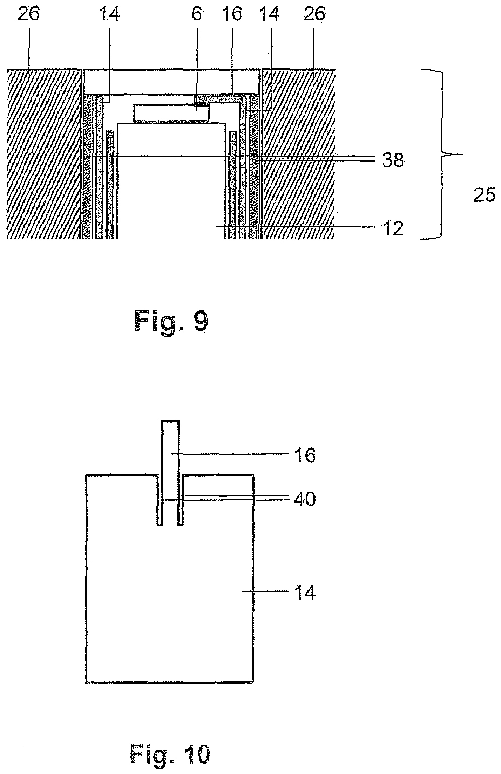

Fig. 9 zeigt eine weitere prinzipielle Ausführungsform, welche auch in Kombination mit den vorangehend beschriebenen Ausführungsformen Verwendung finden könnte. Bei dieser Ausführungsform ist zwischen der Wandung 26 des Endoskopköpfchens 25 und der Hülse 14 eine Hülse 38 aus einem thermisch isolierenden Material angeordnet. Diese Hülse 38 erhöht den thermischen Widerstand in radialer Richtung, um einen Erwärmung des Endoskopköpfchens 25 und insbesondere der Wandung 26 zu minimieren. So wird sichergestellt, dass der Großteil der Verlustwärme in proximaler Richtung über den ersten Leiter 12 abgeführt wird. In Kombination mit der Ausführungsform gemäß Fig. 8 wäre eine elektrische Verbindung zwischen der Wandung 26 und der Hülse 14 durch die Hülse 38 hindurch sicherzustellen. Dies könnte zum einen durch elektrisch leitende Eigenschaften der Hülse 38 oder elektrische Leiter, welche sich durch die Hülse 38 hindurch erstrecken, erreicht werden. Auch könnten Ausnehmungen in der Hülse 38 ausgebildet sein, welche einen elektrischen Kontakt durch die Hülse hindurch ermöglichen.

Die Fig. 10 - 12 zeigen mögliche Ausgestaltungen der Hülse 14, wobei dort die Lasche 16 noch nicht in Richtung quer zur Hülse 14 umgebogen ist und sich noch in Längsrichtung der Umfangswandung der Hülse erstreckt. Die drei Ausführungsformen gemäß Fig. 10 - 12 unterscheiden sich in der Ausgestaltung der Lasche 16.

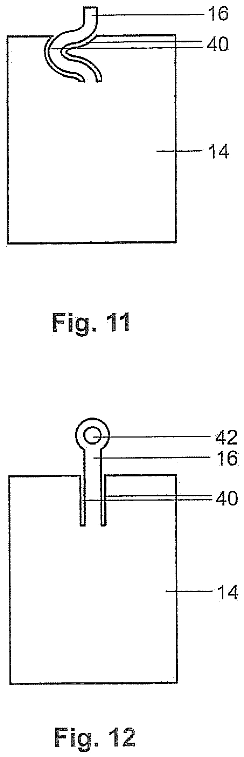

Um eine gewisse Beweglichkeit bzw. Elastizität zwischen LED-Chip 6 und der Hülse 14 zu gewährleisten, wird eine Beweglichkeit der Lasche 16 gewünscht. Dies wird durch schlitzförmige Einschnitte 40 vom distalen Ende her in die Hülse 14 hinein gewährleistet, welche die Lasche 16 begrenzen. So wird die Lasche 16 in die Hülse hinein verlängert und erhält eine größere Beweglichkeit, um mechanische Spannungen zu vermeiden. Durch den bogen- bzw. mäanderförmigen Verlauf der Einschnitte 40 gemäß der Ausführungsform in Fig. 11 wird eine noch größere Beweglichkeit erreicht, insbesondere eine Federwirkung in axialer Richtung erzielt.

In Fig. 12 ist darüber hinaus gezeigt, dass das freie Ende der Lasche 16 eine Kontaktfläche mit einem innenliegenden Loch 42 aufweist. Eine solche Ausgestaltung könnte auch bei den Ausführungsformen gemäß Fig. 10 und 11 Verwendung finden. Das Loch 42 dient als Reservoir für Lötzinn beim Auflöten der Lasche 16 auf den Anschlusskontakt 10 des LED-Chips 6. Darüber hinaus wird sichergestellt, dass das Lot nicht zur Seite weglaufen kann und nicht den strahlungsemittierenden Bereich des LED-Chips Überdeckt. Darüber hinaus muss zum Verlöten weniger Energie appliziert werden, es kann beispielsweise ein Laser als Lötwerkzeug Verwendung finden und die Laserstrahlung kann direkt mit dem Lot in dem Loch 42 in Kontakt kommen, d. h. die Lasche 16 überdeckt das Lot der Lötstelle nicht.

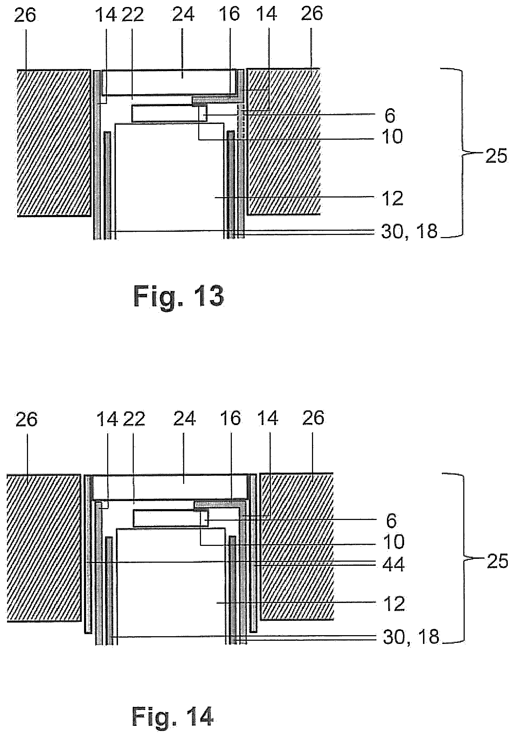

Bei der Ausführungsform gemäß Fig. 13 ist die Hülse 14 über das distale Ende der LED 6 hinaus verlängert, so dass die Hülse 14 ein Fenster 24 aufnehmen kann. Das Fenster 24 ist dicht am Innenumfang der Hülse 14 angeordnet, beispielsweise mit dieser verklebt oder verlötet. Der Freiraum zwischen dem Fenster 24 und der LED 6 bzw. dem ersten elektrischen Leiter 12 ist mit einem transparenten Füllmaterial 22 gefüllt. Am Außenumfang des ersten elektrischen Leiters 12 ist zwischen dem ersten elektrischen Leiter 12 und der Hülse 14 entweder eine erste Isolierung 30 oder der zweite elektrische Leiter 18 angeordnet. Das Fenster 24 sorgt gemeinsam mit dem Füllmaterial 22 und der Hülse 14 dafür, dass das LED-Modul nach außen dicht verschlossen ist und insbesondere die LED 6 mit den elektrischen Anschlusskontakten im Inneren geschützt angeordnet ist. Die Hülse 14 wird außenumfänglich mit der Wandung 26 des medizinischen Instrumentes dicht verbunden.

Fig. 14 zeigt eine weitere Variante, bei welcher das Fenster 24 im Inneren einer Außenhülse 44 angeordnet ist. Das Fenster 24 liegt am Innenumfang der Außenhülse 44 an derem distalen Ende dicht an und ist mit der Außenhülse 44 umfänglich dicht verbunden, beispielsweise lötet oder verklebt. Die Außenhülse 44 erstreckt sich über eine gewisse axiale Länge außenumfänglich der Hülse 14, vorzugsweise so, dass sie sich bis in den Umfangsbereich des ersten elektrischen Leiters 12 hinein erstreckt. So dient die Außenhülse 44 dem Schutz des distalen Endes des LED-Beleuchtungsmoduls und verschließt dieses dicht. Gegenüber der in Fig. 13 gezeigten Ausführungsform hat diese Ausgestaltung den Vorteil, dass der Freiraum im Inneren der Hülse 14, welcher die LED umgibt, zunächst vom distalen Ende her mit dem Füllmaterial 22 gefüllt werden kann und dann die Hülse 14 vom distalen Ende durch Überstülpen der Außenhülse 44 mit dem Fenster 24 verschlossen werden kann. Auch das Verlöten der Lasche 16 mit dem Anschlusskontakt 10 an der Distalseite der LED kann so durch das offene Ende der Hülse 14 vor dem Einfüllen des Füllmaterials 22 geschehen. Die Außenhülse 44 ist dicht mit der Hülse 14 verbunden, beispielsweise verlötet oder verklebt. Gegenüber der in Fig. 13 gezeigten Ausführungsform hat diese Ausgestaltung den Vorteil, dass außenumfänglich keine Öffnungen in dem LED-Modul verbleiben. Bei der Ausführungsform gemäß Fig. 13 verbleibt in dem Bereich, in welchem die Lasche 16 aus der Hülse 14 nach innen gebogen ist, eine Öffnung.

Im Übrigen sind die LED-Module gemäß Fig. 13 und 14 wie die vorangehend beschriebenen LED-Module ausgebildet, so dass auf deren Beschreibung verwiesen wird.

Fig. 15 zeigt eine Schnittansicht eines LED-Beleuchtungsmoduls entsprechend der Ausführungsform gemäß Fig. 4. Sie unterscheidet sich von der Ausführungsform gemäß Fig. 4 darin, dass die Hülse 14 nicht vollumfänglich ausgebildet ist, sondern einen sich axial erstreckenden Schlitz 46 aufweist. Dieser ermöglicht es, die Hülse 14 so auszubilden, dass sie federnd auf die innenliegenden Bauteile wie die Isolierung 30 und den zweiten Leiter 18 aufgeklemmt werden kann. Dazu wird die Hülse 14 so ausgebildet, dass sie in ihrem Ausgangszustand einen geringfügig kleineren Innendurchmesser als der Außendurchmesser der innenliegenden Bauteile aufweist, so dass die Hülse 14 beim Aufsetzen auf die Isolierung 30 bzw. den zweiten elektrischen Leiter 18 elastisch aufgeweitet wird und so durch Federwirkung geklemmt wird. Im Übrigen wird auf die Beschreibung der Ausführungsform gemäß Fig. 4 verwiesen.

### Bezugszeichenliste

2 - LED-Beleuchtungsmodul
4 - distales Ende
6 - LED-Chip
8 - erster Anschlusskontakt
10 - zweiter Anschlusskontakt
12 - erster elektrischer Leiter
14 - Hülse
16 - Lasche
18, 18'- zweiter elektrischer Leiter
20 - Energiequelle
22 - Freiraum
24 - Fenster
25 - Endoskopköpfchen
26 - Wandung
28 - Endoskopschaft
30, 30'- erste Isolierung
32 - zweite Isolierung
34, 34'- Kabel
36 - Sackloch
38 - Hülse
40 - Einschnitte
42 - Loch
44 - Außenhülse
46 - Schlitz

## Patentansprüche

1. Medizinisches Instrument mit einem LED-Beleuchtungsmodul mit zumindest einer LED (6), welche an einer Proximalseite einen ersten elektrischen Anschlusskontakt (8) und an einer axial entgegengesetzt gerichteten Distalseite einen zweiten elektrischen Anschlusskontakt (10) sowie einen Strahlung emittierenden Abstrahlbereich aufweist, **dadurch gekennzeichnet, dass** die LED (6) an ihrem zweiten elektrischen Anschlusskontakt (10) über eine elektrisch leitende Hülse (14) kontaktiert ist, welche beabstandet von ihrem proximalen Ende mit dem zweiten Anschlusskontakt (10) der LED (6) elektrisch leitend verbunden ist und in axialer Richtung eine größere Länge als die LED (6) aufweist, so dass die Hülse als elektrischer Leiter bis mindestens zur Proximalseite der LED wirkt.

2. Medizinisches Instrument nach Anspruch 1, **dadurch gekennzeichnet, dass** die Hülse (14) die LED (6) und/oder einen zu dem ersten elektrischen Anschlusskontakt (8) führenden ersten elektrischen Leiter (12), insbesondere einen diesen isolierenden Mantel umfänglich umgibt.

3. Medizinisches Instrument nach Anspruch 1 oder 2, **dadurch gekennzeichnet, dass** die Hülse (14) die LED (6) und/oder den ersten elektrischen Leiter (12), insbesondere einen diesen isolierenden Mantel vollumfänglich oder teilumfänglich umgibt.

4. Medizinisches Instrument nach einem der vorangehenden Ansprüche, **dadurch gekennzeichnet, dass** die Hülse (14) im Bereich ihres distalen Endes mit dem zweiten Anschlusskontakt (10) der LED (6) elektrisch leitend verbunden ist.

5. Medizinisches Instrument nach einem der vorangehenden Ansprüche, **dadurch gekennzeichnet, dass** die Hülse (14) eine radial nach innen gerichtete Lasche (16) aufweist, welche mit dem zweiten Anschlusskontakt (10) der LED (6) elektrisch leitend verbunden ist.

6. Medizinisches Instrument nach einem der vorangehenden Ansprüche, **dadurch gekennzeichnet, dass** der erste elektrische Anschlusskontakt (8) der LED (6) direkt mit einem ersten elektrischen Leiter (12) zum elektrischen Anschluss der LED (6) verbunden ist.

7. Medizinisches Instrument nach einem der vorangehenden Ansprüche, **dadurch gekennzeichnet, dass** die Hülse (14) an ihrem proximalen Ende oder in ihrem Umfangsbereich direkt oder indirekt mit einem zweiten elektrischen Leiter (18, 18') zum elektrischen Anschluss der LED (6) verbunden ist.

8. Medizinisches Instrument nach einem der vorangehenden Ansprüche, **dadurch gekennzeichnet, dass** der erste elektrische Anschlusskontakt (8) mit einer Gegenkontaktfläche des ersten elektrischen Leiters (12) zum elektrischen Anschluss der LED (6) elektrisch leitend verbunden ist, wobei die Gegenkontaktfläche eine gleiche oder größere Fläche als der erste Anschlusskontakt (8) aufweist.

9. Medizinisches Instrument nach Anspruch 8, **dadurch gekennzeichnet, dass** der erste Anschlusskontakt (8) eine rechteckige oder quadratische Kontaktfläche aufweist und die Gegenkontaktfläche eine runde Form aufweist, wobei der Durchmesser der Gegenkontaktfläche vorzugsweise zumindest der Länge einer Diagonalen der rechteckigen oder quadratischen Kontaktfläche (8) entspricht.

10. Medizinisches Instrument nach einem der vorangehenden Ansprüche, **dadurch gekennzeichnet, dass** ein Koaxialkabel zum elektrischen Anschluss der LED (6) vorgesehen ist, von welchem ein Innenleiter einen ersten elektrischen Leiter (12) bildet, welcher mit dem ersten Anschlusskontakt (8) der LED (6) verbunden ist, und ein Außenleiter (18) elektrisch leitend mit der Hülse (14) verbunden ist.

11. Medizinisches Instrument nach einem der vorangehenden Ansprüche, **dadurch gekennzeichnet, dass** der die LED (6) umgebende Innenraum (22) der Hülse (14) mit einem vorzugsweise für eine von der LED (6) emittierte Strahlung transparenten Füllmaterial gefüllt ist.

12. Medizinisches Instrument nach einem der vorangehenden Ansprüche, **dadurch gekennzeichnet, dass** die Hülse (14) an ihrem Außenumfang von einem thermisch isolierenden Mantel (38) umgeben ist.

13. Medizinisches Instrument nach einem der vorangehenden Ansprüche, **dadurch gekennzeichnet, dass** ein distales Ende der Hülse (14) von der Distalseite der LED (6) in proximaler Richtung beabstandet ist.

14. Medizinisches Instrument nach einem der Ansprüche 1 bis 12, **dadurch gekennzeichnet, dass** die Hülse (14) sich axial über die Distalseite der LED (6) hinaus erstreckt und am distalen Ende der Hülse (14) im Inneren der Hülse (14) ein Fenster (24) angeordnet ist.

15. Medizinisches Instrument nach einem der Ansprüche 1 bis 13, **dadurch gekennzeichnet, dass** die Hülse (14) von einer Außenhülse (44) umgeben ist, welche sich axial über ein distales Ende der Hülse (14) hinaus erstreckt und in derem Inneren am distalen Ende ein Fenster (24) angeordnet ist.

16. Medizinisches Instrument nach einem der vorhergehenden Ansprüche, **dadurch gekennzeichnet, dass** es ein Endoskop ist.

17. Medizinisches Instrument nach Anspruch 16, **dadurch gekennzeichnet, dass** die Hülse (14) des LED-Beleuchtungsmodules (2) mit ihrer distalen Stirnseite an zumindest einer Anschlagfläche (24) im Inneren eines Kopfes (25) des Endoskops anliegt.

## Claims

1. A medical instrument with an LED illumination module with at least one LED (6) which at a proximal side comprises a first electrical connection contact (8) and at an axially oppositely directed distal side comprises a second electrical connection contact (10) as well as a radiation region which emits radiation, **characterised in that** the LED (6) at its second electrical connection contact (10) is connected via an electrically conductive sleeve (14) which, in a manner distanced to its proximal end, is electrically conductively connected to the second connection contact (10) of the LED (6) and in the axial direction has a greater length than the LED (6), so that the sleeve acts as an electrical conductor at least up to the proximal side of the LED.

2. A medical instrument according to claim 1, **characterised in that** the sleeve (14) surrounds the LED (6) and/or a first electrical conductor (12) which leads to the first electrical connection contact (8), in particular surrounds a sheath which insulates this first electrical conductor.

3. A medical instrument according to claim 1 or 2, **characterised in that** the sleeve (14) surrounds the LED (6) and/or the first electrical conductor (12), in particular a sheath which insulates this first electrical conductor, in a fully peripheral or partially peripheral manner.

4. A medical instrument according to one of the preceding claims, **characterised in that** the sleeve (14) in the region of its distal end is electrically conductively connected to the second connection contact (10) of the LED (6).

5. A medical instrument according to one of the preceding claims, **characterised in that** the sleeve (14) comprises a radially inwardly directed tab (16) which is electrically conductively connected to the second connection contact (10) of the LED (6).

6. A medical instrument according to one of the preceding claims, **characterised in that** the first electrical connection contact (8) of the LED (6) is connected in a direct manner to a first electrical conductor (12) for the electrical connection of the LED (6).

7. A medical instrument according to one of the preceding claims, **characterised in that** the sleeve (14) at its proximal end or in its peripheral region is directly or indirectly connected to a second electrical conductor (18, 18') for the electrical connection of the LED (6).

8. A medical instrument according to one of the preceding claims, **characterised in that** the first electrical connection contact (8) is electrically conductively connected to a counter-contact surface of the first electrical conductor (12) for the electrical connection of the LED (6), wherein the counter-contact surface has an equal or larger surface than the first connection contact (8).

9. A medical instrument according to claim 8, **characterised in that** the first connection contact (8) has a rectangular or square contact surface and the counter-contact surface has a round shape, wherein the diameter of the counter-contact surface preferably corresponds at least to the length of a diagonal of the rectangular or square contact surface (8).

10. A medical instrument according to one of the preceding claims, **characterised in that** a coaxial cable for the electrical connection of the LED (6) is provided, of which coaxial cable an inner conductor forms a first electrical conductor (12) which is connected to the first connection contact (8) of the LED (6), and an outer conductor (18) is electrically conductively connected to the sleeve (14).

11. A medical instrument according to one of the preceding claims, **characterised in that** the inner space (22) of the sleeve (14) which surrounds the LED (6) is filled with a filling material which is preferably transparent to a radiation emitted by the LED (6)

12. A medical instrument according to one of the preceding claims, **characterised in that** the sleeve (14) at its outer periphery is surrounded by a thermally insulating sheath (38).

13. A medical instrument according to one of the preceding claims, **characterised in that** a distal end of the sleeve (14) is distanced to the distal side of the LED (6) in the proximal direction.

14. A medical instrument according to one of the claims 1 to 12, **characterised in that** the sleeve (14) extends axially beyond the distal side of the LED (6), and a window (24) is arranged at the distal end of the sleeve (14) in the inside of the sleeve (14).

15. A medical instrument according to one of the claims 1 to 13, **characterised in that** the sleeve (14) is surrounded by an outer sleeve (44) which extends axially beyond a distal end of the sleeve (14), and a window (24) is arranged in the inside of the sleeve at the distal end.

16. A medical instrument according to one of the preceding claims, **characterised in that** it is an endoscope.

17. A medical instrument according to claim 16, **characterised in that** the sleeve (14) of the LED illumination module (2) bears with its distal face side on at least one stop surface (24) in the inside of a head (25) of the endoscope.

## Revendications

1. Instrument médical comportant un module d'éclairage à LED ayant au moins une LED (6) qui présente un premier contact de raccordement électrique (8) sur un côté proximal et un second contact de raccordement électrique (10) sur un côté distal opposé en direction axiale, ainsi qu'une zone de rayonnement émettant un rayonnement, **caractérisé en ce que** la LED (6) est mise en contact au niveau de son second contact de raccordement électrique (10) par l'intermédiaire d'une douille électriquement conductrice (14), qui est connectée de manière électriquement conductrice à distance de son extrémité proximale au second contact de raccordement (10) de la LED (6), et qui présente dans sa direction axiale une plus grande longueur que la LED (6), de sorte que la douille agit comme un conducteur électrique au moins jusqu'au côté proximal de la LED.

2. Instrument médical selon la revendication 1, **caractérisé en ce que** la douille (14) entoure circonférentiellement la LED (6) et/ou un premier conducteur électrique (12) menant au premier contact de raccordement électrique (8), en particulier une gaine isolant celui-ci.

3. Instrument médical selon la revendication 1 ou 2, **caractérisé en ce que** la douille (14) entoure la LED (6) et/ou le premier conducteur électrique (12), en particulier une gaine isolant celui-ci, sur toute sa circonférence ou une partie de celle-ci.

4. Instrument médical selon l'une quelconque des revendications précédentes, **caractérisé en ce que** la douille (14) est reliée de manière électriquement conductrice au second contact de raccordement (10) de la LED (6) dans la zone de son extrémité distale.

5. Instrument médical selon l'une quelconque des revendications précédentes, **caractérisé en ce que** la douille (14) présente une languette (16) orientée radialement vers l'intérieur, qui est reliée de manière électriquement conductrice au second contact de raccordement (10) de la LED (6).

6. Instrument médical selon l'une quelconque des revendications précédentes, **caractérisé en ce que** le premier contact de raccordement électrique (8) de la LED (6) est relié directement à un premier conducteur électrique (12) pour le raccordement électrique de la LED (6).

7. Instrument médical selon l'une quelconque des revendications précédentes, **caractérisé en ce que** la douille (14), à son extrémité proximale ou dans sa zone circonférentielle, est reliée directement ou indirectement à un second conducteur électrique (18, 18') pour le raccordement électrique de la LED (6).

8. Instrument médical selon l'une quelconque des revendications précédentes, **caractérisé en ce que** le premier contact de raccordement électrique (8) est relié de manière électriquement conductrice à une surface de contact antagoniste du premier conducteur électrique (12) pour le raccordement électrique de la LED (6), la surface de contact antagoniste présentant une surface égale ou supérieure au premier contact de raccordement (8).

9. Instrument médical selon la revendication 8, **caractérisé en ce que** le premier contact de raccordement (8) présente une surface de contact rectangulaire ou carrée, et la surface de contact antagoniste présente une forme ronde, le diamètre de la surface de contact antagoniste correspondant, de préférence, au moins à la longueur d'une diagonale de la surface de contact rectangulaire ou carrée (8).

10. Instrument médical selon l'une quelconque des revendications précédentes, **caractérisé en ce qu'**est prévu un câble coaxial pour le raccordement électrique de la LED (6), dont un conducteur intérieur forme un premier conducteur électrique (12) qui est relié au premier contact de raccordement (8) de la LED (6), et un conducteur extérieur (18) est relié de manière électriquement conductrice à la douille (14).

11. Instrument médical selon l'une quelconque des revendications précédentes, **caractérisé en ce que** l'espace intérieur (22) de la douille (14) entourant la LED (6) est rempli d'un matériau de remplissage de préférence transparent pour un rayonnement émis par la LED (6).

12. Instrument médical selon l'une quelconque des revendications précédentes, **caractérisé en ce que** la douille (14) est entourée d'une gaine thermiquement isolante (38) au niveau de sa circonférence extérieure.

13. Instrument médical selon l'une quelconque des revendications précédentes, **caractérisé en ce qu'**une extrémité distale de la douille (14) est distante du côté distal de la LED (6) dans la direction proximale.

14. Instrument médical selon l'une quelconque des revendications 1 à 12, **caractérisé en ce que** la douille (14) s'étend axialement au-delà du côté distal de la LED (6), et une fenêtre (24) est agencée à l'extrémité distale de la douille (14) à l'intérieur de la douille (14).

15. Instrument médical selon l'une quelconque des revendications 1 à 13, **caractérisé en ce que** la douille (14) est entourée d'une douille extérieure (44) qui s'étend axialement au-delà d'une extrémité distale de la douille (14), et à l'intérieur de laquelle une fenêtre (24) est agencée à l'extrémité distale.

16. Instrument médical selon l'une quelconque des revendications précédentes, **caractérisé en ce qu'**il s'agit d'un endoscope.

17. Instrument médical selon la revendication 16, **caractérisé en ce que** la douille (14) du module d'éclairage à LED (2) repose par son côté avant distal sur au moins une surface de butée (24) à l'intérieur d'une tête (25) de l'endoscope.
